(19) Europäisches Patentamt
European Patent Office
Office européen des brevets

(11) Publication number: 0 227 157 B1

(12) EUROPEAN PATENT SPECIFICATION

(45) Date of publication of the patent specification: 11.04.90

(51) Int. Cl.⁴: A01N 47/34, A01N 47/38, C07C 275/62, C07D 233/38, C07D 239/10

(21) Application number: 86202149.0

(22) Date of filing: 02.12.86

(54) **Herbicides.**

(30) Priority: 03.12.85 GB 8529797

(43) Date of publication of application: 01.07.87 Bulletin 87/27

(45) Publication of the grant of the patent: 11.04.90 Bulletin 90/15

(84) Designated Contracting States: AT BE CH DE ES FR GB GR IT LI LU NL SE

(56) References cited:
DE-A- 2 035 796
DE-A- 3 012 123
DE-A- 3 012 124
DE-A- 3 012 190
GB-A- 819 853
US-A- 3 189 431

CHEMICAL ABSTRACTS, vol. 100, no. 20, 14th May 1984, page 536, abstract no. 156604u, Columbus, Ohio, US; & JP-A-58 206 569 (OTSUKA PHARMACEUTICAL CO. LTD) 01.12.1983

(73) Proprietor: SHELL INTERNATIONALE RESEARCH MAATSCHAPPIJ B.V., Carel van Bylandtlaan 30, NL-2596 HR Den Haag(NL)

(72) Inventor: Barker, Michael Derek, 40 Borden Lane, Sittingbourne Kent(GB)
Inventor: Hunter, David Calum, 34 Berkeley Court, Sittingbourne Kent(GB)
Inventor: Smith, Mark Vincent, 155 Park Road, Sittingbourne Kent(GB)
Inventor: Clifford, Ronald David, 55 Imperial Drive Warden Bay, Sheppey Kent(GB)

(74) Representative: Bennett, David Arthur Horder et al, 4, York Road, London SE1 7NA(GB)

EP 0 227 157 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a composition for and a method of controlling undesired plant growth and to compounds for use in such a composition or method.

West German Auslegeschriften 3 012 123 and 3 012 124 relate to certain 1,1,3,5-substituted biuret compounds and Auslegeschrift 3 012 190 relates to certain 1,3,5-substituted biuret compounds and specifically disclose phenyl alkyl biuret compounds, in which the phenyl moiety is monosubstituted by a halogen atom, and pharmaceutical preparations comprising them. The biuret compounds are described as being useful as analgesic, anti-inflammatory and anti-pyretic agents; there is no suggestion that any of the compounds have herbicidal activity, nor that the preparations disclosed would be useful as herbicides.

US Patent Specification No. 3 189 431 discloses the herbicidal activity of phenyl alkyl biurets, including a variety of halophenyl biurets, stating that such substituted biurets have proven to be extremely effective as pre-emergence herbicides. Applicants found that halophenyl alkyl biurets specifically disclosed (e.g. the 2,4-dichlorophenyl, 2,4,5-trichlorophenyl, 3-chlorophenyl, 4-chlorophenyl, 4-bromophenyl and and 4-fluorophenyl compounds (see columns 5 and 6) show very little pre-emergence activity, and only moderate post-emergence activity. However, in spite of this low activity of such halophenyl biurets, Applicants unexpectedly discovered surprising levels of herbicidal activity, including in many cases significant pre-emergence activity, in those halophenyl alkyl biurets bearing a fluorine or chlorine substituent at the 2-position and either no other substituents or certain specific substituents at the 5 position only. This activity appears, surprisingly, to belong uniquely to this specific substitution pattern, since it has been found to be absent from analogous compounds having the halogen compound at either the 3 or 4 position, or having a 2 substituent and also a 4 substituent. (The locant numbering being with reference to the attachment of the phenyl ring to the biuret chain.)

Accordingly, the present invention provides a method of combating undesired plant growth at a locus which comprises treating the locus with a halophenyl alkyl biuret of the general formula I:

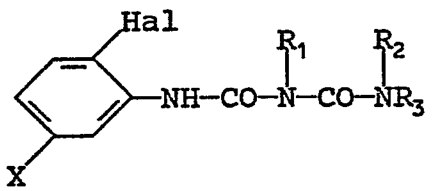

(I)

wherein Hal represents a chlorine or, especially a fluorine atom;

$R_1$ represents a hydrogen atom or a $C_{1-6}$ alkoxy group, preferably $C_{1-4}$, for example methoxy) or, especially, a $C_{1-6}$ alkyl group (preferably $C_{1-4}$, for example methyl);

$R_2$ represents a $C_{1-6}$ alkyl group (preferably $C_{1-4}$, for example methyl) or, especially, a hydrogen atom;

$R_3$ represents a $C_{1-6}$ alkyl group (preferably $C_{1-4}$, for example ethyl or, especially, methyl);

or $R_1$ and $R_3$ together represent a $C_{2-4}$ alkylene group (suitably ethylene or n-propylene);

and X represents a hydrogen atom, a halogen (suitably fluorine, chlorine or iodine) atom; a $C_{7-14}$ aralkyl group (suitably benzyl) or $C_{1-10}$ alkyl group (preferably $C_{1-6}$, for example ethyl, propyl, butyl or pentyl), each of which may optionally be substituted by one or more halogen atoms (suitably chlorine or, especially, fluorine), or by a $C_{1-6}$ alkoxy group (preferably $C_{1-4}$, for example methoxy, ethoxy or propyloxy), a $C_{1-6}$ alkoxyimino group (preferably $C_{1-4}$, for example methoxyimino), a $C_{6-10}$ arylthio group (suitably phenylthio), a $C_{1-6}$ alkylthio group preferably $C_{1-4}$, for example methylthio), a $C_{7-14}$ aralkyloxy group (suitably benzyloxy), a $C_{2-7}$ alkanoyloxy group (preferably $C_{2-5}$, for example acetoxy) or a $C_{3-6}$ acetal group (suitably ethylene acetal); or represents a $C_{6-10}$ aryl (suitably phenyl), a $C_{1-6}$ alkoxy (preferably $C_{1-4}$, for example methoxy or propyloxy), a $C_{7-14}$ aralkyloxy (suitably benzyloxy), $C_{2-6}$ alkenyl (preferably $C_{2-4}$, for example vinyl), ($C_{1-6}$ alkyl)carbamoyl (preferably $C_{1-4}$, mono or dialkyl, for example diethylcarbamoyl), $C_{2-7}$ alkanoyl (preferably $C_{2-5}$, for example acetyl), or $C_{7-11}$ aroyl (suitably benzoyl) group, or a $C_{3-8}$ cycloalkyl group (preferably $C_{3-6}$, for example cyclopropyl or cyclohexyl) optionally substituted by one or more halogen, especially chlorine, atoms, or with a herbicidal composition such a compound in association with a carrier.

Application to the locus may be pre-emergence or post-emergence. The dosage of active ingredient used may, for example, be from 0.05 to 10 kg/ha, preferably from 0.1 to 4 kg/ha.

The present invention also provides the use as a herbicide of a compound of general formula I.

Carbon chains in groups above may be linear or branched.

Preferably X represents a hydrogen or halogen atom, a $C_{1-4}$ alkyl group optionally substituted by one or more fluorine atoms or by a $C_{1-4}$ alkoxy, $C_{1-4}$ alkoxyimino, ethylene acetal or acetoxy group; or a benzyl group optionally substituted in the alkyl moiety by a $C_{1-4}$ alkoxy or $C_{1-4}$ alkoxyimino group; or a phenyl, acetyl, $C_{2-4}$ alkenyl, $C_{1-4}$ alkylcarbamoyl or $C_{1-4}$ alkoxy group; or a $C_{3-6}$ cycloalkyl group, optionally substituted by one or more halogen atoms.

Preferably $R_1$ represents a $C_{1-4}$ alkyl group, for example methyl, $R_2$ represents a $C_{1-4}$ alkyl group, for example methyl, or, more preferably, a hydrogen atom, and $R_3$ represents a $C_{1-4}$ alkyl group, for example methyl. Thus, 1,3-dialkyl biurets, especially 1,3-dimethyl biurets, are especially preferred.

A class of compounds of particularly advantageous pre-emergence activity may be defined in accordance with formula I above, wherein Hal represents chlorine or, especially, fluorine; $R_1$ represents hydrogen, $C_{1-4}$ alkoxy or $C_{1-4}$ alkyl; $R_2$ represents hydrogen or $C_{1-4}$ alkyl; $R_3$ represents $C_{1-4}$ alkyl; and X represents hydrogen, fluorine or iodine, $C_{1-4}$ alkyl optionally substituted by one or more fluorine atoms or by $C_{1-4}$ alkoxy or $C_{1-4}$ alkoxyimino; benzyl optionally substituted in the alkyl moiety by $C_{1-4}$ alkoxy; $C_{3-6}$ cycloalkyl optionally substituted by one or two halogen atoms; $C_{1-4}$ alkoxy; and ($C_{1-4}$ alkylcarbamoyl. In particular, excellent pre-emergence activity is shown by such compounds in which X represents a hydrogen atom, a methyl, ethyl or propyl group optionally substituted by a methoxy, ethoxy or propyloxy group; or a methoxy, propyloxy or benzyloxy group. Preferred alkoxyalkyl groups are ($C_{1-3}$ alkoxy)$C_{2-3}$ alkyl groups, for example 1-methoxyethyl, 1-ethoxyethyl, 1-isopropyloxyethyl, 1-methoxy(n-propyl) and 1-ethoxy(n-propyl).

Especially preferred for good pre-emergence activity are 1,3-dimethyl biurets substituted at the 5-position with a 2'-fluorophenyl group bearing at the 5'-position a hydrogen atom or a ($C_{1-2}$ alkoxy) $C_{2-3}$ alkyl group. The locant numbering adopted hereinafter applies 1, 3 and 5 to denote the respective nitrogen atoms of the biuret chain, with the substitution positions on the phenyl ring being distinguished by a prime superscript and numbered from the attachment of the phenyl ring to the biuret chain.

Many of the compounds are novel, and the invention therefore also extends to these novel compounds per se and to herbicidal compositions comprising said compounds, as active ingredient, and a carrier. The novel compounds are the halophenyl alkyl biurets of formula I as defined above, with the exception of those compounds wherein X is a hydrogen atom. Preferred novel compounds are those wherein the various substituents have the meanings indicated above as preferred in relation to the defined compositions. Individual compounds of particular interest are those wherein Hal represents a fluorine atom, $R_1$ and $R_3$ each represents a methyl group, $R_2$ represents a hydrogen atom, and X represents an isopropyl, trifluoromethyl, or alpha methoxybenzyl group.

The invention also provides a process for the preparation of compounds of the general formula I, which comprises reacting a phenyl isocyanate of formula II:

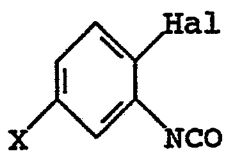

II

with a substituted urea of formula III:

$R_1NHCON(R_2)(R_3)$ III

the substituents Hal, X, $R_1$, $R_2$ and $R_3$ having the meanings defined above in relation to formula I. Suitably the reaction is carried out by heating, eg under reflux, in an inert organic solvent, such as toluene.

When the desired phenyl isocyanate of formula II is not readily available it may conveniently be generated, suitably insitu by reacting phosgene ($COCl_2$) with the appropriate aniline of formula IV:

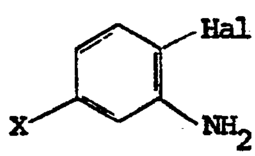

IV

When the desired aniline of formula IV is not readily available it may be generated by standard hydrogenation, (for example catalytic hydrogenation employing a platinum catalyst, for example platinum dioxide, and molecular hydrogen, suitably in an organic solvent, for example ethanol) of the corresponding substituted nitrobenzene. The nitro group may be introduced into the substituted benzene ring by standard techniques. We have found that the use of acetyl nitrate is particularly suitable because it gives a high yield of the desired isomer, in which the nitro group is ortho to the group 'Hal'. In a preferred nitration method acetic anhydride and concentrated nitric acid are mixed at ambient temperature and immediately cooled, for example to a temperature in the range -25°C to -10°C. The substituted benzene compound to be nitrated, cooled to a temperature within the same range, is then added whilst the temperature is maintained within the range.

The preparation of various compounds I wherein X represents a substituted alkyl group may be achieved via an addition reaction of a 2-halo-5-alkenyl nitrobenzene precursor; and, of course, further synthetic routes to useful precursors may then be available, by displacement reactions.

In the particular case where $R_1$ and $R_3$ are methyl and $R_2$ is hydrogen, another convenient alternative is to react the aniline of formula IV with 1,3-dimethyldiazetidinedione, or 3,5-dimethyloxadiazinetrione, suitably by heating, for example at a temperature in the range 30°C to the reflux temperature, preferably from 70 to 90°C, in glacial acetic acid.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitol, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing ½-10% w of active ingredient. Granules are usually prepared to have a size between 10 and 100 BS mesh (1.676 - 0.152 mm), and may be manufactured by agglomeration or impregnation techniques. Generally, granules will contain ½-75% w active ingredient and 0-10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

The composition of the invention may also contain other ingredients, for example other compounds possessing herbicidal, insecticidal or fungicidal properties.

Example 1 1-(2'-Fluorophenyl)-3,5-dimethyl biuret

Equimolar amounts of 2-fluorophenylisocyanate and 1,3-dimethylurea were mixed in toluene and heated under reflux for 5 hours with stirring. On cooling, the desired product crystallised as a solid, m.pt. 144-145°C.
Analysis Calc. C 53.3%; H 5.3; N 18.7%
Found C 53.3%; H 5.5; N 18.7%

Example 2 1-(5'-Benzyl-2'-fluorophenyl)-3,5-dimethyl biuret.

Equimolar amounts of 5-benzyl-2-fluoroaniline and 1,3-dimethyldiazetidinedione were mixed in glacial acetic acid and heated to 80°C for 18 hours with stirring. The mixture was cooled, poured into saturated sodium bicarbonate solution and extracted with ethyl acetate. After drying and evaporation, the crude product was purified by column chromatography on silica gel using ether as eluant to yield the desired product as an oil.
Analysis Calc. C 64.8; H 5.3; N 13.3%
Found C 63.7; H 6.0; N 13.5%
N.M.R. Spectral Data:
2.85 d, 3.23 s, 3.92 s, 5.67-6.17 broad singlet, 6.5-7.43 m, 7.93 d, 11.08 s.

Example 3 1-(5'-(1-Methoxyethyl)-2'-fluorophenyl)-3,5-dimethyl biuret

Methyl(3-nitro-4-fluorophenyl)carbinol (2.86g), trimethyl-oxonium tetrafluoroborate (2.29g) 1,8-bis(dimethylamino)-naphthalene (4g) and dichloromethane (10ml) were mixed, forming a slurry, and stirred at room temperature for 3 days.

Solid material was removed by filtration, washed with dichloromethane, the solvent evaporated and the resulting residue purified by column chromatography on silica using 20% (v/v) diethyl ether/petroleum ether as eluent, yielding 1.8g of 2-fluoro-5-(1-methoxyethyl)nitrobenzene.

Conversion to 2-fluoro-5-(1-methoxyethyl)aniline was then effected by catalytic hydrogenation, as described above.

2-Fluoro-5-(1-methoxyethyl)aniline (2.87g) was then mixed with 3,5-dimethyloxadiazinetrione (2.69g), sodium acetate (0.14g), and glacial acetic acid (30 ml). The reaction mixture was stirred for 3 days at 80°C. The mixture was cooled and poured into saturated sodium bicarbonate solution, extracted with ethyl acetate and purified by column chromatography on silica using 50/50 (v/v) diethylether/petroleum ether as eluant. Yield of the title compound was 1.32g.
Nmr (ppm)1.28d 2.67d 3.05s 3.13s Calc. C 55.1 H 6.4 N 14.8
4.08q 6.33q 6.73s 6.85dd Found C 54.9 H 6.6 N 13.7
7.87dd 11.38d

Following procedures similar to those described, further examples of compounds of the invention were prepared whose physical characteristics and elemental analyses are set out in Table 1 below. Where the compounds were obtained as oils, their identity was confirmed by n.m.r. analysis. In the Table, the compounds are identified by reference to the substituents in general formula I:

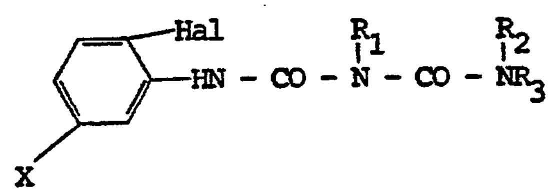

TABLE 1

| Ex. No. | Hal | $R_1$ | $R_2$ | $R_3$ | X | M.Pt./n.m.r. (°C/ppm) | | Analysis C | H | N |
|---|---|---|---|---|---|---|---|---|---|---|
| 4 | F | $CH_3$ | H | $CH_3$ | $C_2H_5$ | 1.20t 2.58q 2.80d 3.22s 6.0-6.5bs 6.70-7.30m 7.82d 11.17s | Calc. | 56.9 | 6.3 | 16.6 |
| | | | | | | | Found | 56.6 | 6.4 | 16.0 |
| 5 | Cl | $CH_3$ | H | $CH_3$ | H | 147-150 | Calc. | 49.7 | 5.0 | 17.4 |
| | | | | | | | Found | 50.15 | 5.05 | 17.3 |
| 6 | F | $CH_3$ | H | $CH_3$ | F | 199-200 | Calc. | 49.4 | 4.6 | 17.3 |
| | | | | | | | Found | 49.4 | 4.9 | 17.6 |
| 7 | F | $CH_3$ | H | $CH_3$ | $C_3H_7{}^i$ | 95-97 | Calc. | 58.4 | 6.8 | 15.7 |
| | | | | | | | Found | 58.5 | 6.8 | 15.5 |
| 8 | F | $CH_3$ | H | $CH_3$ | $CF_3$ | 100-101 | Calc. | 45.1 | 3.8 | 14.3 |
| | | | | | | | Found | 44.3 | 4.1 | 13.9 |
| 9 | F | $CH_3$ | H | $CH_3$ | $C(CH_3)=NOCH_3$ | 147-149 | Calc. | 52.7 | 5.7 | 18.9 |
| | | | | | | | Found | 53.1 | 5.8 | 18.6 |
| 10 | F | $CH_3$ | H | $CH_3$ | $CH=CH_2$ | 2.83d 3.23s 5.27d 5.52d 5.80-6.30bs 6.40-7.30m 8.13d 11.13d | Calc. | 57.4 | 5.6 | 16.7 |
| | | | | | | | Found | 57.6 | 6.0 | 16.4 |

TABLE 1 (continued)

| Ex. No. | Hal | $R_1$ | $R_2$ | $R_3$ | X | M.Pt./n.m.r. | | Analysis C | H | N |
|---|---|---|---|---|---|---|---|---|---|---|
| 11 | F | $CH_3$ | H | $CH_3$ | $CH(CH_3)Cl$ | 1.52d 2.40d 3.32s 5.8q | Calc. | 50.1 | 5.2 | 14.6 |
| | | | | | | 6.12q 6.93d 7.05d 8.20d 11.40s | Found | 53.3 | 6.3 | 14.4 |
| 12 | F | $CH_3$ | H | $CH_3$ | $CH(OCH_3)Ph$ | 2.77d 3.15s 3.30s 5.13s 6.03 q | Calc. | 62.6 | 5.8 | 12.2 |
| | | | | | | 6.80-7.40m 8.07d 11.32d | Found | 61.6 | 6.0 | 11.2 |
| 13 | F | $CH_3$ | H | $CH_3$ | $CON(C_2H_5)_2$ | 164-167 | Calc. | 55.6 | 6.0 | 17.3 |
| | | | | | | | Found | 56.3 | 6.3 | 17.5 |
| 14 | F | $CH_3$ | H | $CH_3$ | I | 124-126 | Calc. | 34.2 | 3.1 | 12.0 |
| | | | | | | | Found | 35.2 | 3.3 | 12.0 |
| 15 | F | $CH_3$ | H | $CH_3$ | $-OCH_2Ph$ | 149-150 | Calc. | 61.6 | 5.5 | 12.7 |
| | | | | | | | Found | 61.6 | 5.5 | 12.8 |
| 16 | F | $CH_3$ | H | $CH_3$ | $OCH(CH_3)_2$ | 140-141 | Calc. | 55.1 | 6.4 | 14.8 |
| | | | | | | | Found | 55.4 | 6.4 | 14.9 |
| 17 | F | $CH_3$ | H | $C_2H_5$ | H | 91-3 | Calc. | 55.2 | 5.9 | 17.6 |
| | | | | | | | Found | 55.3 | 6.0 | 16.8 |
| 18 | F | $CH_3$ | $CH_3$ | $CH_3$ | H | 2.88s 3.13s 6.6-7.2m | Calc. | 55.2 | 5.9 | 17.7 |
| | | | | | | 7.8-8.2m 9.88 broad s | Found | 54.9 | 5.9 | 17.6 |

TABLE 1 (continued)

| Ex. No. | Hal | $R_1$ | $R_2$ | $R_3$ | X | M.Pt./n.m.r. | Analysis | C | H | N |
|---|---|---|---|---|---|---|---|---|---|---|
| 19 | F | H | H | $CH_3$ | H | 154-6 | Calc. | 51.2 | 4.7 | 19.9 |
| | | | | | | | Found | 50.9 | 4.85 | 20.05 |
| 20 | F | H | $CH_3$ | $CH_3$ | H | 175.5-177.5 | Calc. | 53.3 | 5.4 | 18.7 |
| | | | | | | | Found | 53.7 | 5.3 | 18.6 |
| 21 | F | H | $CH_3$ | $CH_3$ | $CH(CH_3)OCH_3$ | 101-3 | Calc. | 55.1 | 6.4 | 14.8 |
| | | | | | | | Found | 57.5 | 6.9 | 13.5 |
| 22 | F | $CH_3$ | H | $CH_3$ | 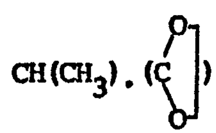 | 1.60s 2.87d 3.28s 3.60-4.10m 5.7-6.1bs 7.08t 7.45-7.75m 8.22dd 11.47s | Calc. | 54.0 | 5.8 | 13.5 |
| | | | | | | | Found | 54.6 | 5.5 | 14.4 |
| 23 | F | $CH_3$ | H | $CH_3$ | $COCH_3$ | 2.63s 2.95d 3.37s 5.7-6.1bs 7.10dd 7.50-7.80m 8.92dd 11.57s | Calc. | 53.9 | 5.2 | 15.7 |
| | | | | | | | Found | 54.5 | 5.6 | 15.1 |
| 24 | F | $CH_3$ | H | $CH_3$ | COPh | 174 | Calc. | 62.0 | 4.9 | 12.8 |
| | | | | | | | Found | 61.5 | 5.0 | 12.7 |
| 25 | F | $CH_3$ | H | $CH_3$ | 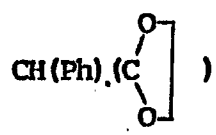 | 2.62d 3.05s 3.93s 6.0-7.8m 8.15d 11.80d | Calc. | 61.1 | 5.4 | 11.3 |
| | | | | | | | Found | 61.8 | 5.7 | 10.7 |

TABLE 1 (continued

| Ex. No. | Hal | $R_1$ | $R_2$ | $R_3$ | X | M.Pt./n.m.r. | | Analysis C | H | N |
|---|---|---|---|---|---|---|---|---|---|---|
| 26 | F | $CH_3$ | H | $CH_3$ | $C(Ph)=NOCH_3$ | 2.8d 3.18s 3.9s 6.0b 7.17m 8.07m | Calc. | 60.3 | 5.3 | 15.6 |
| | | | | | | 11.4b | Found | 59.9 | 5.6 | 15.7 |
| 27 | F | $CH_3$ | H | $CH_3$ | $CH(CH_3)(OC_2H_5)$ | 1.17t 1.4d 2.88d 3.30s 3.38q | Calc. | 56.6 | 6.7 | 14.1 |
| | | | | | | 4.38q 6.17b 7.05m 8.07m 11.17b | Found | 55.8 | 6.8 | 12.9 |
| 28 | F | $CH_3$ | H | $CH_3$ | Ph | 149-153 | Calc. | 63.8 | 5.3 | 14.0 |
| | | | | | | | Found | 63.6 | 5.4 | 13.6 |
| 29 | F | $CH_3$ | H | $CH_3$ | $OCH_3$ | 166-168 | Calc. | 51.8 | 5.5 | 16.5 |
| | | | | | | | Found | 51.5 | 5.6 | 16.3 |
| 30 | F | $CH_3$ | H | $CH_3$ | $CH(CH_3)(SPh)$ | 1.55d 2.80d 3.20s 4.30q 6.07b | Calc. | 59.8 | 5.5 | 11.6 |
| | | | | | | 7.175m 8.13m 11.07b | Found | 61.5 | 5.7 | 10.4 |
| 31 | F | $CH_3$ | H | $CH_3$ | 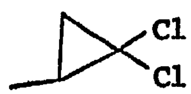 | 1.83dd 2.57m 2.8d 3.23s 6.22b | Calc. | 46.7 | 4.2 | 12.6 |
| | | | | | | 7.0m 8.03m 11.4b | Found | 49.3 | 5.0 | 12.2 |
| 32 | F | $CH_3$ | H | $CH_3$ | $n-C_3H_7$ | 92-93 | Calc. | 58.4 | 6.7 | 15.7 |
| | | | | | | | Found | 58.45 | 6.75 | 15.4 |
| 33 | F | $CH_3$ | H | $CH_3$ | $-CH(CH_3)(SCH_3)$ | 1.58d 1.93s 2.90d 3.33s 6.375b | Calc. | 52.2 | 6.0 | 14.0 |
| | | | | | | 7.07m N1.33b 8.18m | Found | 52.3 | 6.2 | 13.7 |

TABLE 1 (continued)

| Ex. No. | Hal | $R_1$ | $R_2$ | $R_3$ | X | M.Pt./n.m.r. | | Anaylsis C | H | N |
|---|---|---|---|---|---|---|---|---|---|---|
| 34 | F | $CH_3$ | H | $CH_3$ | $-CH(CH_3)$ | 1.12d 1.15d 1.38d 2.87d 3.30s | Calc. | 57.9 | 7.1 | 13.5 |
| | | | | | $O-iC_3H_9$ | 3.43s septet 4.53q 6.33b 7.07m | Found | 57.4 | 6.9 | 14.0 |
| | | | | | | 8.035m 11.23b | | | | |
| 35 | F | $CH_3$ | H | $CH_3$ | $-CH(CH_3)$ | 1.45d 2.8d 3.23s 4.15m 6.25b | Calc. | 63.5 | 6.1 | 11.7 |
| | | | | | $OCH_2Ph$ | 7.17m 8.08m 11.27b | Found | 63.4 | 6.3 | 10.4 |
| 36 | F | $CH_3$ | H | $CH_3$ | $CH(C_2H_5)(OCH_3)$ | 79-81 | Calc. | 56.6 | 6.7 | 14.15 |
| | | | | | | | Found | 56.25 | 6.8 | 13.95 |
| 37 | F | $CH_3$ | H | $CH_3$ | $CH(C_2H_5)$ | 71-73 | Calc. | 57.0 | 7.05 | 13.5 |
| | | | | | $OC_2H_5$ | | Found | 57.9 | 7.1 | 13.35 |
| 38 | F | $CH_3$ | H | $CH_3$ | $-CH(C_2H_5)_2$ | 88.5-90 | Calc. | 61.0 | 7.45 | 14.25 |
| | | | | | | | Found | 61.1 | 7.55 | 14.2 |
| 39 | F | H | $CH_3$ | $CH_3$ | $-CH(CH_3)$ | 1.17t 1.25d 3.1s 3.37q 6.05q | Calc. | 56.6 | 6.7 | 14.1 |
| | | | | | $OC_2H_5$ | 7.0m 8.12m 9.03b 9.6b | Found | 56.5 | 6.8 | 14.1 |
| 40 | F | H | $CH_3$ | $CH_3$ | $-C(CH_3)=NOCH_3$ | 2.17s 3.0s 3.87s 7.08m 8.28m 8.6b | | | | |
| | | | | | | 11.17b | | | | |

TABLE 1 (continued)

| Ex. No. | Hal | $R_1$ | $R_2$ | $R_3$ | X | M.Pt./n.m.r. | Anaylsis | C | H | N |
|---|---|---|---|---|---|---|---|---|---|---|
| 41 | F | H | $CH_3$ | $CH_3$ | $C_2H_5$ | 1.28t 2.83q 3.25s 7.17m 8.05m 8.57b 11.6b | | | | |
| 42 | F | H | $CH_3$ | $CH_3$ | F | 205.5-206.5 | Calc. | 49.4 | 4.6 | 17.3 |
| | | | | | | | Found | 49.5 | 4.2 | 16.9 |
| 43 | F | $OCH_3$ | H | $CH_3$ | H | 149.5-152 | Calc. | 49.8 | 5.0 | 17.4 |
| | | | | | | | Found | 49.2 | 5.1 | 17.2 |
| 44 | F | $(R_1+R_3) = -(CH_2)_2-$ | $CH_3$ | | H | 162-164 | Calc. | 55.7 | 5.1 | 17.7 |
| | | | | | | | Found | 55.7 | 5.1 | 17.7 |
| 45 | F | $(R_1+R_3) = -(CH_2)_3-$ | $CH_3$ | | H | 106-108 | Calc. | 57.4 | 5.6 | 16.7 |
| | | | | | | | Found | 57.4 | 5.7 | 16.91 |
| 46 | Cl | $CH_3$ | H | $CH_3$ | Cl | 160-162 | Calc. | 43.5 | 4.0 | 15.2 |
| | | | | | | | Found | 43.9 | 4.1 | 15.1 |
| 47 | Cl | $CH_3$ | H | $CH_3$ | $C_2H_5$ | 121-123 | Calc. | 53.4 | 6.0 | 15.6 |
| | | | | | | | Found | 53.5 | 6.0 | 15.6 |
| 48 | F | $CH_3$ | H | $CH_3$ | $CH(CH_3)O.COCH_3$ | 1.43d 1.95s 2.68d 3.12s | Calc. | 54.0 | 5.8 | 13.5 |
| | | | | | | 5.63q 6.45b 6.83b 7.98b 11.58d | Found | 54.4 | 6.1 | 12.7 |

TABLE 1 (continued)

| Ex. No. | Hal | $R_1$ | $R_2$ | $R_3$ | X | M.Pt./n.m.r. | | Anaylsis C | H | N |
|---|---|---|---|---|---|---|---|---|---|---|
| 49 | F | $CH_3$ | H | $CH_3$ | 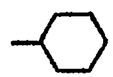 | 137.5-139 | Calc. | 62.5 | 7.2 | 13.7 |
| | | | | | | | Found | 62.6 | 7.3 | 13.8 |
| 50 | F | $CH_3$ | H | $CH_3$ | Sec-$C_4H_9$ | 81.5-82.5 | Calc. | 59.8 | 7.1 | 14.95 |
| | | | | | | | Found | 59.2 | 7.05 | 14.65 |

Example 51 1-(5'-Tert-butyl-2'-fluorophenyl)-3,5-dimethyl biuret

Fuming nitric acid (specific gravity 1.5; 1.1ml) was added dropwise to stirred acetic anhydride (3ml) at 25°C. The mixture was stirred for 1 minute then cooled to -20°C. p-Tert-butylfluorobenzene (2g) in acetic anhydride (2ml) was stirred in a 25 ml round bottomed flask and cooled to -20°C. the acetyl nitrate was added dropwise and the reaction temperature was maintained below -15°C. Work up was effected by adding the reaction mixture to ice/water and extracting with ethyl acetate.

The product, comprising 2-fluoro-5-tert-butylnitrobenzene, was then hydrogenated, and then converted to the title compound by the method of Example 2. m.p.138-140°C.

Analysis: Calc.C59.8 H7.2 N14.9
Found C 60.1 H7.3 N14.7

Example 52

Herbicidal Activity

To evaluate their herbicidal activity, compounds according to the invention were tested using as representative range of plants: maize, Zea mays (Mz); rice, Oryza sativa (R); barnyard grass, Echinochloa crusgalli (BG); oat, Avena sativa (O); linseed, Linum usitatissimum (L); mustard, Sinapsis alba (M); sugar beet, Beta vulgaris (SB) and soya bean, Glycine max (S).

The tests fall into two categories, pre-emergence and post-emergence. The pre-emergence tests involved spraying a liquid formulation of the compound onto the soil in which the seeds of the plant specied mentioned above had recently been sown. The post-emergence tests involved two types of test, viz., soil drench and foliar spray tests. In the soil drench tests the soil in which the seedling plants of the above species were growing was drenched with a liquid formulation containing a compound of the invention, and in the foliar spray tests the seedling plants were sprayed with such a formulation.

The soil used in the tests was a prepared horticultural loam.

The formulations used in the tests were prepared from solutions of the test compounds in acetone containing 0.4% by weight of an alkylphenol/ethylene oxide condensate available under the trade mark TRITON X-155. These acetone solutions were diluted with water and the resulting formulations applied at dosage levels corresponding to 5 kg or 1 kg of active material per hectare in a volume equivalent to 600 litres per hectare in the soil spray and foliar spray test, and at a dosage of level equivalent to 10 kilograms of active material per hectare in a volume equivalent to approximately 3,000 litres per hectare in the soil drench tests.

In the pre-emergence tests untreated sown soil and in the post-emergence tests untreated soil bearing seedling plants were used as controls.

The herbicidal effects of the test compounds were assessed visually twelve days after spraying the foliage and the soil, and thirteen days after drenching the soil and were recorded on a 0-9 scale. A rating 0 indicates growth as untreated control, a rating 9 indicates death. An increase of 1 unit on the linear scale approximates to a 10% increase in the level of effect. A blank space indicates that testing was not effected for example because there was insufficient compound for all tests.

The results of the tests are set out in Table 2 below, in which the compounds are identified by reference to the preceding examples.

TABLE 2

| Compound of Ex. No. | Soil drench 10/kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 5 | 9 | 9 | 8 | 9 | 9 | 9 | 9 | 7 | 5 | 6 | 5 | 9 | 8 | 9 | 9 | 9 | 8 | 3 | 4 | 9 | 7 | 8 | 9 | 9 | 6 |
| | | | | | | | | | 1 | 3 | 2 | 3 | 5 | 6 | 9 | 8 | 4 | 1 | 2 | 3 | 5 | 7 | 7 | 7 | 0 |
| 1 | 7 | 8 | 9 | 8 | 9 | 9 | 9 | 9 | 5 | 8 | 5 | 9 | 9 | 9 | 9 | 9 | 9 | 4 | 4 | 9 | 7 | 7 | 9 | 9 | 6 |
| | | | | | | | | | 1 | 6 | 3 | 9 | 8 | 9 | 9 | 9 | 9 | 2 | 0 | 9 | 7 | 4 | 9 | 9 | 3 |
| 6 | 6 | 6 | 9 | 8 | 6 | 9 | 9 | 8 | 5 | 2 | 5 | 9 | 8 | 8 | 9 | 9 | 8 | 3 | 0 | 7 | 4 | 4 | 9 | 8 | 0 |
| | | | | | | | | | 1 | 1 | 3 | 8 | 7 | 8 | 9 | 9 | 7 | 2 | 0 | 7 | 3 | 4 | 7 | 8 | 0 |
| 7 | 3 | 7 | 5 | 9 | 9 | 9 | 9 | 9 | 5 | 7 | 6 | 8 | 8 | 9 | 9 | 9 | 9 | 0 | 4 | 7 | 8 | 8 | 9 | 9 | 5 |
| | | | | | | | | | 1 | 4 | 3 | 7 | 7 | 8 | 9 | 9 | 9 | 0 | 0 | 3 | 4 | 5 | 8 | 8 | 3 |
| 8 | 3 | 8 | 7 | 9 | 9 | 9 | 9 | 6 | 5 | 5 | 5 | 8 | 9 | 9 | 9 | 9 | 8 | 2 | 2 | 8 | 4 | 8 | 9 | 9 | 2 |
| | | | | | | | | | 1 | 3 | 2 | 7 | 8 | 8 | 9 | 9 | 7 | 0 | 0 | 2 | 2 | 6 | 7 | 9 | 1 |

TABLE 2 (continued)

| Compound of | | | Soil drench 10/kg/ha | | | | | | Dosage | | | Foliar spray | | | | | | | | Pre-emergence | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 3 | 7 | 8 | 9 | 9 | 9 | 9 | 9 | 9 | 5 | 6 | 8 | 9 | 8 | 8 | 9 | 9 | 8 | 3 | 5 | 9 | 7 | 8 | 9 | 9 | 6 |
| | | | | | | | | | 1 | 3 | 6 | 8 | 7 | 8 | 9 | 9 | 6 | 3 | 4 | 9 | 7 | 8 | 9 | 9 | 4 |
| 9 | 3 | 7 | 8 | 9 | 9 | 9 | 9 | 7 | 5 | 3 | 7 | 9 | 8 | 9 | 9 | 9 | 9 | 3 | 2 | 8 | 7 | 6 | 9 | 8 | 3 |
| | | | | | | | | | 1 | 1 | 3 | 7 | 6 | 8 | 9 | 9 | 9 | 0 | 0 | 4 | 6 | 4 | 8 | 8 | 2 |
| 4 | 5 | 8 | 8 | 9 | 8 | 9 | 9 | 7 | 5 | 1 | 6 | 9 | 8 | 9 | 9 | 9 | 9 | 3 | 2 | 9 | 7 | 6 | 9 | 8 | 2 |
| | | | | | | | | | 1 | 1 | 2 | 8 | 7 | 9 | 9 | 9 | 8 | 0 | 0 | 5 | 5 | 2 | 7 | 8 | 0 |
| 10 | 0 | 6 | 9 | 9 | 9 | 9 | 9 | 7 | 5 | 4 | 1 | 9 | 6 | 9 | 9 | 9 | 9 | 0 | 0 | 0 | 5 | 4 | 9 | 8 | 4 |
| | | | | | | | | | 1 | 0 | 1 | 8 | 6 | 9 | 9 | 9 | 7 | 0 | 0 | 0 | 0 | 2 | 8 | 4 | 1 |
| 11 | | | | | | | | | 5 | 3 | 0 | 7 | 5 | 7 | 9 | 9 | 8 | 0 | 0 | 4 | 4 | 3 | 9 | 6 | 4 |
| | | | | | | | | | 1 | 0 | 0 | 5 | 3 | 4 | 8 | 7 | 5 | 0 | 0 | 0 | 0 | 1 | 6 | 1 | 1 |

TABLE 2 (continued)

| Compound of Ex. No. | Soil drench 10/kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 2 | 0 | 0 | 0 | 1 | 0 | 0 | 6 | 4 | 5 | 4 | 0 | 8 | 7 | 9 | 9 | 9 | 8 | 0 | 0 | 0 | 0 | 4 | 9 | 9 | 3 |
| | | | | | | | | | 1 | 2 | 0 | 7 | 6 | 9 | 9 | 9 | 7 | 0 | 0 | 0 | 0 | 2 | 9 | 8 | 1 |
| 12 | 4 | 3 | 7 | 7 | 6 | 9 | 8 | 5 | 5 | 4 | 3 | 8 | 7 | 9 | 9 | 9 | 8 | 2 | 0 | 5 | 3 | 5 | 8 | 9 | 2 |
| | | | | | | | | | 1 | 4 | 1 | 6 | 5 | 9 | 9 | 9 | 7 | 1 | 0 | 2 | 1 | 2 | 8 | 7 | 0 |
| 13 | 5 | 8 | 9 | 8 | 9 | 9 | 9 | 9 | 5 | 4 | 5 | 5 | 7 | 9 | 9 | 9 | 9 | 3 | 0 | 9 | 7 | 7 | 9 | 9 | 5 |
| | | | | | | | | | 1 | 1 | 3 | 5 | 4 | 6 | 7 | 9 | 8 | 0 | 0 | 7 | 6 | 5 | 7 | 8 | 1 |
| 14 | 4 | 8 | 5 | 9 | 9 | 9 | 9 | 7 | 5 | 6 | 4 | 9 | 8 | 9 | 9 | 9 | 9 | 0 | 4 | 9 | 6 | 7 | 9 | 9 | 5 |
| | | | | | | | | | 1 | 4 | 2 | 8 | 6 | 8 | 9 | 9 | 8 | 0 | 0 | 2 | 2 | 4 | 9 | 9 | 3 |
| 17 | 5 | 7 | 8 | 8 | 9 | 9 | 9 | 9 | 5 | 4 | 6 | 9 | 7 | 8 | 9 | 9 | 9 | 0 | 0 | 8 | 6 | 6 | 9 | 8 | 5 |
| | | | | | | | | | 1 | 0 | 1 | 8 | 2 | 5 | 7 | 6 | 6 | 0 | 0 | 5 | 5 | 5 | 8 | 8 | 1 |

TABLE 2 (continued)

| Compound of Ex. No. | Soil drench 10/kg/ha | | | | | | | | Dosage kg/ha | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 18 | 6 | 8 | 9 | 9 | 9 | 9 | 9 | 9 | 5 | 5 | 7 | 9 | 8 | 9 | 9 | 9 | 9 | 0 | 4 | 9 | 7 | 7 | 9 | 8 | 7 |
| | | | | | | | | | 1 | 0 | 4 | 8 | 5 | 6 | 9 | 9 | 8 | 0 | 0 | 6 | 6 | 6 | 9 | 8 | 5 |
| 19 | 7 | 8 | 9 | 8 | 9 | 9 | 9 | 9 | 5 | 5 | 5 | 8 | 8 | 9 | 9 | 9 | 8 | 0 | 4 | 5 | 6 | 7 | 8 | 8 | 5 |
| | | | | | | | | | 1 | 2 | 3 | 4 | 6 | 7 | 8 | 8 | 5 | 0 | 1 | 1 | 6 | 4 | 7 | 4 | 1 |
| 20 | 7 | 9 | 9 | 9 | 9 | 9 | 9 | 7 | 5 | 6 | 6 | 9 | 9 | 8 | 9 | 9 | 8 | 4 | 3 | 9 | 8 | 8 | 9 | 8 | 5 |
| | | | | | | | | | 1 | 3 | 2 | 4 | 6 | 6 | 8 | 9 | 5 | 2 | 2 | 7 | 7 | 5 | 8 | 6 | 2 |
| 21 | 6 | 7 | 8 | 9 | 8 | 9 | 9 | 9 | 5 | | | | | | 9 | 9 | 8 | 2 | 4 | 9 | 7 | 8 | 9 | 9 | 7 |
| | | | | | | | | | 1 | 0 | 0 | 6 | 5 | 6 | 9 | 9 | 6 | 1 | 2 | 7 | 6 | 5 | 8 | 9 | 6 |
| 22 | 6 | 7 | 7 | 8 | 9 | 9 | 9 | 9 | 1 | 2 | 1 | 4 | 3 | 5 | 7 | 5 | 4 | 0 | 0 | 0 | 0 | 0 | 0 | 0 | 0 |

TABLE 2 (continued)

| Compound of | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No | Mz | R | BG | O | L | M | SB | S | kg/ha | Mz | R | BG | O | L | M | SB | S | Mz | R | BG | O | L | M | SB | S |
| 23 | 4 | 3 | 5 | 7 | 8 | 8 | 9 | 8 | 5 | 0 | 3 | 7 | 6 | 5 | 9 | 9 | 4 | 0 | 0 | 5 | 5 | 3 | 8 | 5 | 0 |
| | | | | | | | | | 1 | 0 | 2 | 5 | 4 | 3 | 8 | 8 | 2 | 0 | 0 | 1 | 2 | 1 | 7 | 0 | 0 |
| 24 | 0 | 2 | 5 | 2 | 4 | 8 | 6 | 2 | 5 | 0 | 0 | 6 | 5 | 4 | 9 | 9 | 6 | 0 | 0 | 5 | 0 | 0 | 8 | 5 | 0 |
| | | | | | | | | | 1 | 0 | 0 | 3 | 1 | 2 | 8 | 7 | 3 | 0 | 0 | 0 | 0 | 0 | 2 | 0 | 0 |
| 25 | 0 | 1 | 3 | 3 | 6 | 5 | 5 | 2 | 5 | 2 | 2 | 8 | 5 | 9 | 9 | 8 | 9 | 0 | 0 | 0 | 0 | 0 | 5 | 8 | 0 |
| | | | | | | | | | 1 | 0 | 0 | 3 | 2 | 4 | 8 | 6 | 4 | 0 | 0 | 0 | 0 | 0 | 2 | 3 | 0 |
| 26 | 0 | 0 | 0 | 0 | 0 | 5 | 5 | 0 | 5 | 0 | 0 | 8 | 5 | 9 | 9 | 9 | 6 | 0 | 0 | 0 | 0 | 2 | 8 | 3 | 0 |
| | | | | | | | | | 1 | 0 | 0 | 6 | 3 | 7 | 9 | 9 | 4 | 0 | 0 | 0 | 0 | 0 | 4 | 2 | 0 |
| 27 | 6 | 8 | 8 | 9 | 9 | 9 | 9 | 8 | 5 | 5 | 6 | 9 | 6 | 8 | 9 | 9 | 8 | 5 | 3 | 9 | 7 | 7 | 9 | 9 | 7 |
| | | | | | | | | | 1 | 1 | 4 | 8 | 7 | 7 | 9 | 9 | 7 | 3 | 2 | 9 | 7 | 6 | 9 | 9 | 5 |

TABLE 2 (continued)

| Compound of Ex. No. | Soil drench 10/kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 28 | 0 | 0 | 0 | 0 | 0 | 2 | 4 | 2 | 5 | 4 | 3 | 7 | 7 | 9 | 9 | 9 | 5 | 0 | 0 | 0 | 0 | 0 | 5 | 4 | 0 |
| | | | | | | | | | 1 | 2 | 2 | 7 | 6 | 8 | 9 | 9 | 4 | 0 | 0 | 0 | 0 | 0 | 2 | 2 | 0 |
| 29 | 4 | 6 | 9 | 8 | 9 | 9 | 9 | 6 | 5 | 0 | 6 | 9 | 6 | 8 | 9 | 9 | 8 | 3 | 2 | 9 | 6 | 6 | 8 | 8 | 1 |
| | | | | | | | | | 1 | 0 | 3 | 5 | 3 | 7 | 9 | 9 | 5 | 0 | 1 | 3 | 1 | 2 | 7 | 8 | 0 |
| 30 | | | | | | | | | 5 | 3 | 1 | 6 | 4 | 8 | 8 | 9 | 3 | 0 | 0 | 0 | 0 | 0 | 5 | 7 | 0 |
| | | | | | | | | | 1 | 2 | 0 | 4 | 3 | 5 | 6 | 9 | 2 | 0 | 0 | 0 | 0 | 0 | 0 | 2 | 0 |
| 31 | 0 | 3 | 2 | 6 | 7 | 6 | 9 | 3 | 5 | 2 | 3 | 9 | 6 | 9 | 9 | 9 | 8 | 0 | 0 | 4 | 4 | 6 | 9 | 8 | 0 |
| | | | | | | | | | 1 | 1 | 2 | 7 | 6 | 8 | 9 | 9 | 5 | 0 | 0 | 0 | 1 | 9 | 8 | 8 | 0 |
| 32 | 0 | 6 | 6 | 6 | 7 | 9 | 8 | 6 | 5 | 4 | 5 | 9 | 9 | 9 | 9 | 9 | 8 | 3 | 4 | 7 | 6 | 8 | 9 | 9 | 3 |
| | | | | | | | | | 1 | 2 | 3 | 9 | 6 | 8 | 9 | 9 | 8 | 0 | 1 | 1 | 1 | 3 | 8 | 8 | 0 |

TABLE 2 (continued)

| Compound of | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 33 | 2 | 5 | 2 | 3 | 4 | 4 | 7 | 2 | 5 | 3 | 2 | 8 | 6 | 8 | 9 | 8 | 6 | 0 | 0 | 2 | 3 | 0 | 7 | 4 | 2 |
| | | | | | | | | | 1 | 0 | 0 | 5 | 4 | 6 | 8 | 7 | 5 | 0 | 0 | 0 | 0 | 0 | 0 | 1 | 0 |
| 34 | 6 | 6 | 8 | 9 | 9 | 9 | 9 | 8 | 5 | 5 | 7 | 9 | 9 | 9 | 9 | 9 | 9 | 4 | 4 | 9 | 7 | 6 | 9 | 9 | 3 |
| | | | | | | | | | 1 | 3 | 4 | 6 | 6 | 9 | 9 | 9 | 6 | 0 | 2 | 5 | 5 | 5 | 9 | 9 | 0 |
| 35 | 0 | 0 | 0 | 1 | 2 | 5 | 5 | 0 | 5 | 3 | 3 | 5 | 4 | 9 | 9 | 9 | 6 | 0 | 0 | 0 | 0 | 0 | 9 | 8 | 0 |
| | | | | | | | | | 1 | | | 5 | 4 | 8 | 9 | 9 | 6 | 0 | 0 | 0 | 0 | 0 | 5 | 6 | 0 |
| 36 | 9 | 9 | 9 | 9 | 9 | 9 | 9 | 9 | 5 | 7 | 7 | 9 | 9 | 9 | 9 | 9 | 9 | 4 | 5 | 9 | 7 | 8 | 9 | 9 | 5 |
| | | | | | | | | | 1 | 4 | 5 | 8 | 8 | 8 | 9 | 9 | 8 | 1 | 3 | 9 | 7 | 6 | 9 | 9 | 3 |
| 37 | 6 | 9 | 9 | 9 | 6 | 9 | 9 | 7 | 5 | 7 | 8 | 9 | 9 | 9 | 9 | 9 | 9 | 3 | 4 | 9 | 7 | 8 | 9 | 9 | 4 |
| | | | | | | | | | 1 | 3 | 5 | 6 | 8 | 9 | 9 | 9 | 7 | 1 | 2 | 9 | 8 | 5 | 9 | 9 | 0 |

TABLE 2 (continued)

| Compound of Ex. No. | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | kg/ha | Mz | R | BG | O | L | M | SB | S | Mz | R | BG | O | L | M | SB | S |
| 38 | 0 | 0 | 0 | 8 | 5 | 8 | 7 | 2 | 5 | 5 | 5 | 8 | 8 | 8 | 9 | 9 | 8 | 0 | 0 | 8 | 3 | 8 | 8 | 9 | 0 |
| | | | | | | | | | 1 | 3 | 2 | 7 | 6 | 8 | 9 | 8 | 6 | 0 | 0 | 1 | 0 | 2 | 7 | 5 | 0 |
| 39 | 8 | 8 | 9 | 9 | 9 | 9 | 9 | 9 | 5 | 4 | 5 | 9 | 6 | 9 | 9 | 9 | 7 | 4 | 6 | 9 | 8 | 9 | 9 | 9 | 6 |
| | | | | | | | | | 1 | 1 | 9 | 5 | 4 | 5 | 7 | 5 | 5 | 0 | 5 | 9 | 6 | 4 | 9 | 9 | 3 |
| 40 | 3 | 4 | 2 | 7 | 0 | 6 | 9 | 5 | 5 | 2 | 4 | 8 | 6 | 7 | 9 | 9 | 4 | 0 | 0 | 2 | 4 | 3 | 6 | 8 | 2 |
| | | | | | | | | | 1 | 1 | 1 | 6 | 4 | 4 | 6 | 9 | 2 | 0 | 0 | 1 | 3 | 1 | 2 | 7 | 0 |
| 41 | | | | | | | | | 5 | 4 | 4 | 9 | 8 | 8 | 9 | 9 | 8 | 0 | 0 | 8 | 6 | 5 | 8 | 8 | 3 |
| | | | | | | | | | 1 | 2 | 2 | 9 | 6 | 8 | 9 | 9 | 7 | 0 | 0 | 4 | 5 | 2 | 3 | 2 | 0 |
| 42 | 5 | 0 | 6 | 6 | 7 | 9 | 9 | 8 | 5 | 6 | 2 | 8 | 7 | 6 | 7 | 9 | 5 | 0 | 0 | 0 | 0 | 0 | 0 | 0 | 0 |
| | | | | | | | | | 1 | 4 | 1 | 4 | 4 | 4 | 7 | 8 | 4 | 0 | 0 | 0 | 0 | 0 | 0 | 0 | 0 |

TABLE 2 (continued)

| Compound of Ex. No. | Soil drench 10/kg/ha Mz | R | PG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 43 | 5 | 7 | 8 | 9 | 9 | 9 | 9 | 9 | 5 | 3 | 4 | 6 | 5 | 8 | 8 | 9 | 6 | 0 | 3 | 8 | 7 | 8 | 9 | 8 | 4 |
| | | | | | | | | | 1 | 2 | 2 | 1 | 3 | 6 | 6 | 8 | 4 | 0 | 0 | 3 | 4 | 3 | 6 | 6 | 0 |
| 44 | 4 | 6 | 8 | 7 | 9 | 9 | 9 | 8 | 5 | 3 | 4 | 9 | 7 | 7 | 9 | 9 | 9 | 0 | 0 | 8 | 6 | 5 | 8 | 5 | 4 |
| | | | | | | | | | 1 | 1 | 2 | 9 | 5 | 5 | 9 | 9 | 8 | 0 | 0 | 2 | 4 | 0 | 4 | 6 | 1 |
| 45 | 1 | 6 | 6 | 8 | 7 | 9 | 9 | 4 | 5 | 2 | 4 | 9 | 6 | 6 | 9 | 9 | 4 | 1 | 2 | 6 | 6 | 4 | 8 | 8 | 1 |
| | | | | | | | | | 1 | 1 | 1 | 8 | 4 | 3 | 6 | 8 | 1 | 1 | 1 | 2 | 1 | 1 | 3 | 4 | 0 |
| 46 | 0 | 0 | 0 | 4 | 6 | 6 | 7 | 2 | 5 | 3 | 4 | 7 | 6 | 6 | 9 | 9 | 5 | 0 | 0 | 0 | 0 | 0 | 0 | 0 | 0 |
| | | | | | | | | | 1 | 2 | 2 | 7 | 6 | 6 | 8 | 8 | 4 | 0 | 0 | 0 | 0 | 0 | 0 | 0 | 0 |
| 47 | 0 | 5 | 5 | 8 | 7 | 5 | 5 | 9 | 5 | 2 | 6 | 8 | 6 | 9 | 9 | 9 | 6 | 0 | 0 | 3 | 3 | 5 | 7 | 8 | 0 |
| | | | | | | | | | 1 | 0 | 4 | 7 | 5 | 8 | 9 | 9 | 5 | 0 | 0 | 0 | 0 | 3 | 2 | 8 | 0 |

TABLE 2 (continued)

| Compound of Ex. No. | Soil drench 10/kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 15 | 0 | 0 | 0 | 0 | 0 | 0 | 0 | 0 | 5 | 4 | 0 | 6 | 6 | 8 | 9 | 9 | 6 | 0 | 0 | 0 | 0 | 0 | 3 | 3 | 2 |
| | | | | | | | | | 1 | 0 | 0 | 6 | 5 | 7 | 8 | 9 | 6 | 0 | 0 | 0 | 0 | 0 | 0 | 2 | 0 |
| 16 | 4 | 5 | 7 | 7 | 8 | 9 | 9 | 7 | 5 | 3 | | 9 | 7 | 8 | 9 | 9 | 8 | 3 | 2 | 6 | 6 | 6 | 9 | 9 | 5 |
| | | | | | | | | | 1 | 2 | | 8 | 3 | 8 | 9 | 9 | 8 | 1 | 1 | 1 | 4 | 4 | 8 | 9 | 2 |
| 48 | 2 | 6 | 3 | 9 | 9 | 9 | 9 | 9 | 5 | 1 | 0 | 0 | 3 | 2 | 8 | 5 | 4 | 0 | 0 | 0 | 0 | | | | 0 |
| | | | | | | | | | 1 | 0 | 0 | 0 | 3 | 1 | 4 | 3 | 2 | 0 | 0 | 0 | 0 | 0 | 2 | 0 | 0 |

TABLE 2 (continued)

| Compound of | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 49 | 0 | 0 | 0 | 0 | 3 | 0 | 2 | 0 | 5 | 5 | 3 | 8 | 6 | 9 | 9 | 9 | 6 | 0 | 0 | 0 | 0 | 5 | 9 | 9 | 0 |
| | | | | | | | | | 1 | 5 | 2 | 7 | 5 | 9 | 7 | 7 | 4 | 0 | 0 | 0 | 0 | 2 | 9 | 9 | 0 |
| 50 | 0 | 0 | 2 | 6 | 3 | 9 | 9 | 2 | 5 | 2 | 6 | 9 | 8 | 9 | 9 | 9 | 8 | 0 | 0 | 7 | 3 | 6 | 9 | 9 | 1 |
| | | | | | | | | | 1 | 1 | 4 | 9 | 7 | 9 | 9 | 9 | 8 | 0 | 0 | 3 | 1 | 5 | 8 | 8 | 0 |
| 51 | 0 | 2 | 0 | 1 | 6 | 9 | 9 | 2 | 5 | 3 | 4 | 9 | 8 | 9 | 9 | 9 | 6 | 0 | 0 | 0 | 0 | 0 | 8 | 9 | 0 |
| | | | | | | | | | 1 | 2 | 2 | 7 | 6 | 8 | 9 | 9 | 6 | 0 | 0 | 0 | 0 | 0 | 5 | 8 | 0 |

Comparative Herbicidal Evaluation

In order to demonstrate the critical effect of the phenyl substitution pattern on herbicidal activity, the above test procedures were applied to a selection of halophenyl alkyl biurets having the halogen atoms in locations different from those required in the present invention. The results of these comparative tests are set out in Table 3 below, in which the test compounds are identified by reference to the halogen nature/location in the following formula:

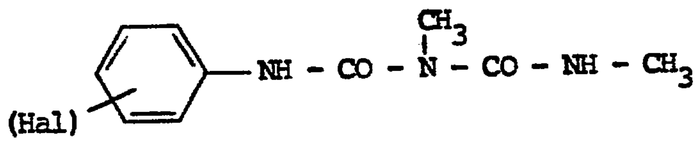

TABLE 3

| | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| HAL | Mz | R | BG | O | L | M | SB | S | kg/ha | Mz | R | BG | O | L | M | SB | S | Mz | R | BG | O | L | M | SB | S |
| 4-Bromo | 0 | 0 | 0 | 0 | 0 | 0 | 0 | 0 | 5 | 0 | 0 | 0 | 0 | 4 | 4 | 6 | 3 | 0 | 0 | 0 | 0 | 0 | 0 | 0 | 0 |
| | | | | | | | | | 1 | 0 | 0 | 0 | 0 | 2 | 3 | 4 | 0 | 0 | 0 | 0 | 0 | 0 | 0 | 0 | 0 |
| 4-Fluoro | 0 | 0 | 0 | 4 | 6 | 7 | 5 | 4 | 5 | 0 | 4 | 6 | 5 | 7 | 6 | 6 | 7 | 0 | 0 | 5 | 2 | 5 | 6 | 5 | 0 |
| | | | | | | | | | 1 | 0 | 0 | 2 | 2 | 4 | 4 | 4 | 3 | 0 | 0 | 2 | 1 | 2 | 3 | 2 | 0 |
| 3-chloro | 6 | 6 | 3 | 7 | 9 | 9 | 5 | 4 | 5 | 0 | 3 | 5 | 5 | 7 | 9 | 6 | 5 | 0 | 0 | 4 | 3 | 5 | 8 | 8 | 0 |
| | | | | | | | | | 1 | 0 | 1 | 2 | 2 | 2 | 4 | 3 | 2 | 0 | 0 | 0 | 0 | 5 | 5 | 7 | 0 |
| 4-chloro | 0 | 0 | 0 | 5 | 6 | 6 | 4 | 4 | 5 | 2 | 2 | 0 | 4 | 5 | 5 | 4 | 4 | 0 | 0 | 0 | 0 | 0 | 0 | 0 | 0 |
| | | | | | | | | | 1 | 1 | 1 | 0 | 2 | 0 | 3 | 3 | 2 | 0 | 0 | 0 | 0 | 0 | 0 | 0 | 0 |

TABLE 3 (continued)

| HAL | Soil drench 10/kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 2,4- | 0 | 0 | 0 | 0 | 3 | 6 | 6 | 0 | 5 | 3 | 4 | 8 | 6 | 5 | 9 | 8 | 6 | 2 | 0 | 0 | 1 | 3 | 8 | 8 | 0 |
| dichloro | | | | | | | | | 1 | 1 | 2 | 5 | 4 | 4 | 7 | 8 | 4 | 0 | 0 | 0 | 0 | 0 | 2 | 3 | 0 |
| 2,4,5- | 0 | 0 | 0 | 0 | 0 | 0 | 0 | 0 | 5 | 0 | 0 | 0 | 1 | 4 | 6 | 6 | 3 | 0 | 0 | 0 | 0 | 0 | 0 | 0 | 0 |
| trichloro | | | | | | | | | 1 | 0 | 0 | 0 | 0 | 2 | 3 | 4 | 1 | 0 | 0 | 0 | 0 | 0 | 0 | 0 | 0 |

## Claims

1. A method of combating undesired plant growth at a locus, which comprises treating the locus with a halophenyl alkyl biuret of general formula I:

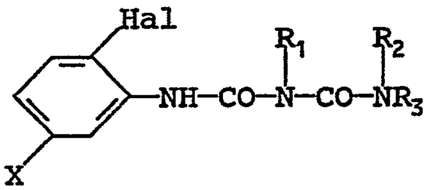

(I)

wherein Hal represents a chlorine or, especially, a fluorine atom;
$R_1$ represents a hydrogen atom, a $C_{1-6}$ alkoxy group, a $C_{1-6}$ alkyl group;
$R_2$ represents a $C_{1-6}$ alkyl group or a hydrogen atom;
$R_3$ represents a $C_{1-6}$ alkyl group;
or $R_1$ and $R_3$ together represent a $C_{2-4}$ alkylene group;
and X represents a hydrogen or halogen atom; a $C_{7-14}$ aralkyl group or $C_{1-10}$ alkyl group, each of which may optionally be substituted by one or more halogen atoms, or by a $C_{1-6}$ alkoxy, $C_{1-6}$ alkoxyimino, $C_{6-10}$ arylthio, $C_{1-6}$ alkylthio, $C_{7-14}$ aralkyloxy, $C_{2-7}$ alkanoyloxy or $C_{3-6}$ acetal group; or a $C_{6-10}$ aryl, $C_{1-6}$ alkoxy, $C_{7-14}$ aralkyloxy, $C_{2-6}$ alkenyl, ($C_{1-6}$ alkyl)carbamoyl, $C_{2-7}$ alkanoyl, or $C_{7-11}$ aroyl group, or a $C_{3-8}$ cycloalkyl group optionally substituted by one or more halogen atoms; or with a herbicidal composition which comprises a carrier and, as active ingredient, the halophenyl alkyl biuret defined above.

2. A method as claimed in Claim 1, wherein in the halophenyl alkyl biuret of formula I, $R_1$ is a methyl group and $R_2$ is a hydrogen atom.

3. A method as claimed in claim 1 or claim 2, wherein, in the halophenyl alkyl biuret of formula I, X represents a hydrogen or halogen atom, a $C_{1-4}$ alkyl group optionally substituted by one or more fluorine atoms or by a $C_{1-4}$ alkoxy, $C_{1-4}$ alkoxyimino, ethylene acetal or acetoxy group; or a benzyl group optionally substituted in the alkyl moiety by a $C_{1-4}$ alkoxy or a $C_{1-4}$ alkoxyimino group; or a phenyl, acetyl, $C_{2-4}$ alkenyl, $C_{1-4}$ alkylcarbamoyl, or $C_{1-4}$ alkoxy group; or a $C_{3-6}$ cycloalkyl group, optionally substituted by one or more halogen atoms.

4. Use as a herbicide of a halophenyl alkyl biuret as defined in any one of claims 1 to 3.

5. A halophenyl alkyl biuret of the formula I as defined in claim 1, wherein the substituents have the meanings defined in claim 1, except that X may not represent a hydrogen atom.

6. A process for the preparation of a compound as defined in claim 5, which comprises reacting a phenyl isocyanate of formula II:

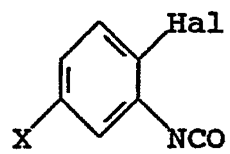

II

with a substituted urea of formula III:

$R_1NHCONR_2(R_3)$ III

the substituents Hal, X, $R_1$, $R_2$ and $R_3$ having the meanings defined in claim 5.

7. A process as claimed in claim 6, wherein the phenyl isocyanate of formula II is generated <u>in situ</u> by reacting phosgene with a amine of formula IV:

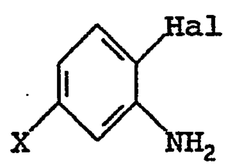

IV

8. A process for the preparation of a compound as claimed in claim 5, wherein $R_1$ and $R_3$ are methyl and $R_2$ is hydrogen, which comprises reacting an amine of formula IV

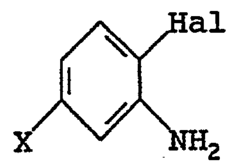

IV

with 1,3-dimethylazetidinedione or 3,5-dimethyloxadiazinetrione.

9. A herbicidal composition which comprises a carrier and, as active ingredient, a halophenyl alkyl biuret as defined in claim 5.

**Patentansprüche**

1. Verfahren zur Bekämpfung von unerwünschtem Pflanzenwuchs an einem Ort, welches ein Behandeln des Ortes mit einem Halogenphenylalkylbiuret der allgemeinen Formel I:

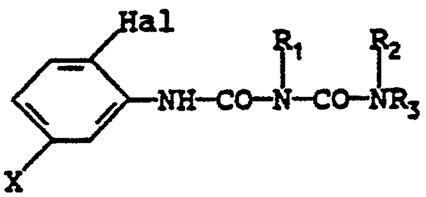

(I),

worin Hal ein Chloratom oder insbesondere ein Fluoratom bedeutet;

$R_1$ ein Wasserstoffatom, eine $C_{1-6}$-Alkoxygruppe oder eine $C_{1-6}$-Alkylgruppe darstellt;

$R_2$ eine $C_{1-6}$-Alkylgruppe oder ein Wasserstoffatom bedeutet;

$R_3$ eine $C_{1-6}$-Alkylgruppe darstellt;

oder $R_1$ und $R_3$ gemeinsam eine $C_{2-4}$-Alkylengruppe bedeuten;

und X ein Wasserstoff- oder Halogenatom; eine $C_{7-14}$-Aralkylgruppe oder $C_{1-10}$-Alkylgruppe, von denen jede gegebenenfalls durch ein oder mehrere Halogenatome oder durch eine $C_{1-6}$-Alkoxygruppe, $C_{1-6}$-Alkoxyiminogruppe, $C_{6-10}$-Arylthiogruppe, $C_{1-6}$-Alkylthiogruppe, $C_{7-14}$-Aralkyloxygruppe, eine $C_{2-7}$-Alkanoyloxygruppe oder eine $C_{3-6}$-Acetalgruppe substituiert sein kann; oder eine $C_{6-10}$-Arylgruppe, $C_{1-6}$-Alkoxygruppe, $C_{7-14}$-Aralkyloxygruppe, $C_{2-6}$-Alkenylgruppe, ($C_{1-6}$-Alkyl)carbamoylgruppe, $C_{2-7}$-Alkanoylgruppe oder $C_{7-11}$-Aroylgruppe oder eine $C_{3-8}$-Cycloalkylgruppe, die gegebenenfalls durch ein oder mehrere Halogenatome substituiert ist, bedeutet;

oder mit einer herbiziden Zusammensetzung, welche einen Träger und als wirksamen Bestandteil das vorstehend definierte Halogenphenylalkylbiuret enthält, umfaßt.

2. Verfahren nach Anspruch 1, worin in dem Halogenphenylalkylbiuret der Formel I $R_1$ eine Methylgruppe bedeutet und $R_2$ ein Wasserstoffatom darstellt.

3. Verfahren nach Anspruch 1 oder 2, worin in dem Halogenphenylalkylbiuret der Formel I X ein Wasserstoff- oder Halogenatom, eine $C_{1-4}$-Alkylgruppe, die gegebenenfalls durch ein oder mehrere Fluoratome oder durch eine $C_{1-4}$-Alkoxygruppe, $C_{1-4}$-Alkoxyiminogruppe, Ethylenacetalgruppe oder Acetoxygruppe substituiert ist; oder eine Benzylgruppe, die gegebenenfalls im Alkylteil durch eine $C_{1-4}$-Alkoxygruppe oder eine $C_{1-4}$-Alkoxyiminogruppe substituiert ist; oder eine Phenylgruppe, Acetylgruppe, $C_{2-4}$-Alkenylgruppe, $C_{1-4}$-Alkylcarbamoylgruppe oder $C_{1-4}$-Alkoxygruppe; oder eine $C_{3-6}$-Cycloalkylgruppe, die gegebenenfalls durch ein oder mehrere Halogenatome substituiert ist, bedeutet.

4. Verwendung eines Halogenphenylalkylbiurets, wie in einem der Ansprüche 1 bis 3 definiert, als ein Herbizid.

5. Ein Halogenphenylalkylbiuret der Formel I, wie in Anspruch 1 definiert, worin die Substituenten die in Anspruch 1 definierten Bedeutungen aufweisen, mit der Ausnahme, daß X nicht ein Wasserstoffatom darstellen kann.

6. Verfahren zur Herstellung einer Verbindung, wie in Anspruch 5 definiert, welches ein Umsetzen eines Phenylisocyanats der Formel II:

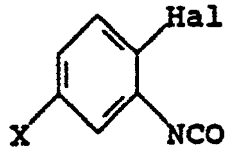

II

mit einem substituierten Harnstoff der Formel III:

$R_1NHCONR_2(R_3)$ III,

wobei die Substituenten Hal, X, $R_1$, $R_2$ und $R_3$ die in Anspruch 5 definierten Bedeutungen aufweisen, umfaßt.

7. Verfahren nach Anspruch 6, worin das Phenylisocyanat der Formel II in situ durch Umsetzen von Phosgen mit einem Amin der Formel IV:

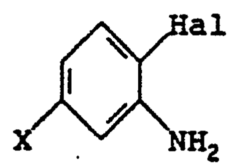

IV

ausgebildet wird.

8. Verfahren zur Herstellung einer Verbindung, wie in Anspruch 5 definiert, worin $R_1$ und $R_3$ Methyl bedeuten und $R_2$ Wasserstoff darstellt, welches Verfahren ein Umsetzen eines Amins der Formel IV:

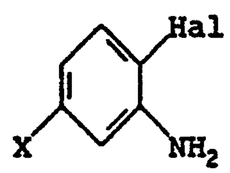

IV

mit 1,3-Dimethylazetidindion oder 3,5-Dimethyloxadiazintrion umfaßt.

9. Herbizide Zusammensetzung, welche einen Träger und als wirksamen Bestandteil ein Halogenphenylalkylbiuret, wie in Anspruch 5 definiert, umfaßt.

**Revendications**

1. Une méthode pour combattre la croissance des plantes indésirables en un lieu, qui consiste à traiter le lieu avec un halophényl-alkyl-biuret de formule générale 1:

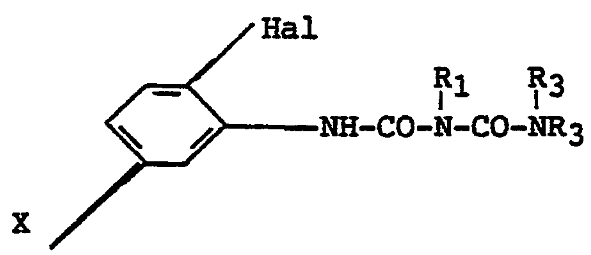

(I)

dans lequel:

Hal représente un atome de chlore ou, spécialement, un atome de fluor;

$R_1$ représente un atome d'hydrogène, un groupe alcoxy en $C_2$ à $C_6$, un groupe alkyle en $C_1$ à $C_6$;

$R_2$ représente un groupe alkyle en $C_1$ à $C_6$ ou un atome d'hydrogène;

$R_3$ représente un groupe alkyle en $C_1$ à $C_6$;

ou bien $R_1$ et $R_3$ représentent un groupe alcylène en $C_2$ à $C_4$;

et X représente un atome d'hydrogène ou un atome d'halogène, un groupe aralkyle en $C_7$ à $C_{14}$ ou un groupe alkyle en $C_1$ à $C_{10}$, chacun d'entre eux pouvant être optionnellement substitué par un ou plus d'un atome d'halogène, ou bien par un groupe alcoxy en $C_1$ à $C_6$, alcoxy-imino en $C_1$ à $C_6$, arylthio en $C_6$ à $C_{10}$, alkylthio en $C_1$ à $C_6$, aralkyloxy en $C_7$ à $C_{14}$, alcanoyloxy en $C_2$ à $C_7$ ou acétal en $C_3$ à $C_6$; ou un groupe aryle en $C_6$ à $C_{10}$, alcoxy en $C_1$ à $C_6$, aralkyloxy en $C_7$ à $C_{14}$, alcényle en $C_2$ à $C_6$ (alkyle en $C_1$ à $C_6$) carbamoyle, alcanoyle en $C_2$ à $C_7$ ou aroyle en $C_7$ à $C_{11}$ ou un groupe cycloalkyle en $C_3$ à $C_8$ optionnellement substitué par un ou plus d'atome d'halogène; ou avec une composition herbicide qui comprend un support et comme ingrédient actif, le halophényl-alkyl-biuret défini ci-dessus.

2. Une méthode telle que revendiquée dans la revendication 1, dans laquelle, dans le halophényl-alkyl-biuret de formule I, $R_1$ est un groupe méthyle et $R_2$ est un atome d'hydrogène.

3. Une méthode telle que revendiquée dans la revendication 1 ou la revendication 2 dans laquelle, dans le halophényl-alkyl-biuret de formule I, X représente un atome d'hydrogène ou d'halogène, un groupe alkyle en $C_1$ à $C_4$ optionnellement substitué par un ou plus d'un atome de fluor ou par un groupe alcoxy en $C_1$ à $C_4$, alcoxyimino en $C_1$ à $C_4$, éthylène-acétal ou acétoxy; ou un groupe benzyle optionnellement

substitué dans la partie alkyle par un groupe alcoxy en $C_1$ à $C_4$ ou un groupe alcoxyimino en $C_1$ à $C_4$; ou un groupe phényle, acétyle, alcényle en $C_2$ à $C_4$, alkylcarbamoyle en $C_1$ à $C_4$ ou alcoxy en $C_1$ à $C_4$; ou un groupe cycloalkyle en $C_3$ à $C_6$ optionnellement substitué par un ou plus d'un atome d'halogène.

4. L'utilisation comme herbicide d'un halophényl-alkyl-biuret tel que défini dans l'une quelconque des revendications 1 à 3.

5. Un halophényl-alkyl-biuret de la formule I tel que défini dans la revendication 1, dans lequel les substituants ont les significations définies dans la revendication 1, sauf que X peut ne pas représenter un atome d'hydrogène.

6. Un procédé pour la préparation d'un composé tel que défini dans la revendication 5, qui consiste à faire réagir un isocyanate de phényle de formule II:

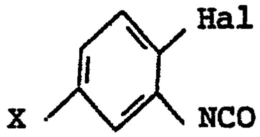

II

avec une urée substituée de formule III:

$$R_1NHCONR_2(R_3) \quad III$$

les substituants Hal, X, $R_1$, $R_2$ et $R_3$ ayant les significations définies dans la revendication 5.

7. Un procédé tel que revendiqué dans la revendication 6, dans lequel l'isocyanate de phényle de formule II est produit <u>in situ</u> par réaction du phosgène avec une amine de formule IV:

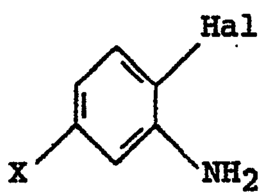

IV

8. Un pocédé pour la préparation d'un composé tel que revendiqué dans la revendication 5, dans lequel $R_1$ et $R_3$ sont du méthyle et $R_2$ est de l'hydrogène, qui consiste à faire réagir une amine de formule IV

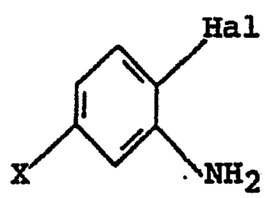

IV

avec de la 1,3-diméthylazetidinedione ou de la 3,5-diméthyloxadiazinetrione.

9. Une composition herbicide qui comprend un support et, comme ingrédient actif, un halophényl-alkyl-biuret tel que défini dans la revendication 5.